(19) Europäisches Patentamt
European Patent Office
Office européen des brevets

(11) **EP 3 165 238 B1**

(12) **EUROPÄISCHE PATENTSCHRIFT**

(45) Veröffentlichungstag und Bekanntmachung des Hinweises auf die Patenterteilung:
**14.02.2018 Patentblatt 2018/07**

(51) Int Cl.:
*A61L 27/04* (2006.01)     *A61L 27/50* (2006.01)
*A61L 31/02* (2006.01)     *A61L 31/18* (2006.01)
*A61B 90/00* (2016.01)

(21) Anmeldenummer: **16187958.0**

(22) Anmeldetag: **09.09.2016**

(54) **RÖNTGENMARKER FÜR EINE ENDOPROTHESE**

X-RAY MARKER FOR AN ENDOPROSTHESIS

MARQUEUR DE RADIOGRAPHIE POUR UNE ENDOPROTHESE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.11.2015 DE 102015118859**

(43) Veröffentlichungstag der Anmeldung:
**10.05.2017 Patentblatt 2017/19**

(73) Patentinhaber: **Biotronik AG**
**8180 Bülach (CH)**

(72) Erfinder: **Bayer, Ullrich**
**18209 Bad Doberan (DE)**

(74) Vertreter: **Randoll, Sören**
**Biotronik SE & Co. KG**
**Corporate Intellectual Property**
**Woermannkehre 1**
**12359 Berlin (DE)**

(56) Entgegenhaltungen:
EP-A2- 2 184 038      EP-A2- 2 399 619
EP-A2- 2 457 601      WO-A2-95/30384
US-A1- 2007 043 429

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft einen Röntgenmarker für eine Endoprothese. Ferner betrifft die Erfindung eine Endoprothese mit einem metallischen Grundkörper und mit einem an dem Grundkörper befestigten Röntgenmarker.

**Technologischer Hintergrund**

Stents sind endovaskuläre Prothesen, die zur Behandlung von Stenosen (Gefäßverengungen) eingesetzt werden können. Sie weisen einen Grundkörper in Form eines hohlzylinder- oder röhrenförmigen Grundgitters auf, das an beiden Längsenden der Röhren offen ist. Das röhrenförmige Grundgitter einer derartigen Endoprothese wird in das zu behandelnde Gefäß eingesetzt und dient dazu, das Gefäß zu stützen.

Derartige Stents oder andere Endoprothesen weisen häufig einen Grundkörper aus einem metallischen Werkstoff auf. Für die vorliegende Erfindung von besonderer Bedeutung sind biodegradierbare metallische Werkstoffe. Unter Biodegradation werden dabei hydrolytische, enzymatische und andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus. Für den Grundkörper biodegradierbarer Endoprothesen geeignete Werkstoffe umfassen beispielsweise Legierungen von Magnesium, Eisen, Zink und Wolfram.

Zur postoperativen Überwachung des Heilungsfortschrittes oder zur Kontrolle minimalinvasiver Eingriffe ist die Röntgendiagnostik ein wichtiges Instrumentarium. So werden beispielsweise seit einigen Jahren Stents in den Koronararterien bei der akuten Myokardinfarkttherapie platziert. Nach gängigen Verfahren wird ein Katheter, der den Stent in einem nicht expandierten Zustand trägt, im Bereich der Läsion der koronaren Gefäßwand positioniert. Anschließend weitet sich der Stent entweder durch selbstexpansive Kräfte oder durch Inflation eines Ballons auf, um im expandierten Zustand eine Obstruktion der Gefäßwand zu verhindern. Der Vorgang der Positionierung und Expansion der Stents muss während der Prozedur durch den Kardiologen laufend überwacht werden.

Im medizinischen Bereich wird mit Röntgenstrahlen im Energiebereich von 60 bis 120 keV, bei Anwendung am Herz typischerweise im Bereich von 80 bis 100 keV gearbeitet. Da der Röntgenabsorptionskoeffizient stark von der Energie abhängt, ist dieser Arbeitsbereich bei der Auswahl von geeigneten Markermaterialien zu berücksichtigen. Die Absorption (Intensitätsabschwächung) der Röntgenstrahlen kann vereinfacht mit einem exponentiellen Abschwächungsgesetz beschrieben werden.

$$\frac{I}{I_0} = \exp\left[-\left(\frac{\mu}{\rho}\right)x\right]$$

Dabei ist I die gemessene Intensität nach dem Probendurchgang, $I_0$ die Intensität der Strahlung vor dem Probendurchgang, $\mu/\rho$ der Massenabsorptionskoeffizient und x die Massendicke der Probe. Für Legierungen berechnet sich der Massenabsorptionskoeffizient durch Aufsummieren der Komponenten.

Bei geringer Absorption des gewählten Werkstoffs in einem gegebenen Energiebereich der Röntgenabsorption könnte demnach durch Erhöhung der Materialdicke eine Verbesserung der Röntgensichtbarkeit erreicht werden; jedoch stößt diese Maßnahme schnell an ihre Grenzen, insbesondere, wenn es um die Markierung filigraner Strukturen, wie sie bei Stents vorliegen, geht.

Es ist somit bekannt, Implantate mit einem Marker in Form einer Beschichtung, eines Bandes, eines Inlays oder andersartig angeformten Design zur Verbesserung der Röntgensichtbarkeit auszustatten. Beispielsweise werden Metallbänder aus Gold oder anderen Edelmetallen in bestimmten Bereichen eines Stents angebracht. Beispiele für derartige Röntgenmarker sind EP 2 184 038 A2, US 2007/0043429 A1 und EP 1 570 808 B1 zu entnehmen.

Bei Implantaten aus biokorrodierbaren metallischen Werkstoffen - zum Beispiel auf der Basis von Magnesium, Eisen, Zink oder Wolfram - bestehen weitere Anforderungen an das Markermaterial:

- der Marker sollte durch die korrosiven Prozesse nicht frühzeitig vom Grundkörper des Implantates getrennt werden, um einer Fragmentbildung und damit der Gefahr der Embolisation vorzubeugen;

- der Marker sollte schon bei geringen Materialdicken eine hinreichende Röntgendichte besitzen; und

- das Markermaterial sollte möglichst keinen oder allenfalls einen geringen Einfluss auf die Degradation des Grundkörpers haben.

Bei Stents, deren Grundkörper aus einem metallischen Material besteht, ergibt sich bei der Anordnung von Röntgenmarkern, beispielsweise bei radioopaken Elementen aus Gold, an dem Stent-Grundkörper das Problem, dass an dem Kontaktbereich zwischen dem Material des Grundkörpers und dem Material des Funktionselements eine Kontaktkorrosion auftritt. Diese führt zur Zerstörung des Stents beziehungsweise zur Abtrennung des Röntgenmarkers von der Stentstruktur, so dass die Endoprothese nicht mehr in der Lage ist, ihre Funktion zu erfüllen, bzw. nicht mehr aufgefunden werden kann. Röntgenmarker aus Gold oder Platin können aufgrund ihrer sehr ausgeprägten Lokalelementwirkung bei Grundkörpern aus Magnesium nicht zur Anwendung kommen. Auch bei Röntgenmarkern aus verschiedenen metallischen Komponenten stellt sich das Problem der Lokalelementbildung.

In der Druckschrift US 6,355,058 B1 wird ein Stent beschrieben, bei dem radioopake Marker in einem polymeren Bindemittel als Partikel eingeschlossen sind. Der Binder ist auf der Oberfläche des Stents ausgebreitet (dispergiert). Eine derartige Verteilung von radioopaken Partikeln bewirkt in der Regel keine ausreichende Dichte dieser Materialien, so dass die Röntgensichtbarkeit für viele Anwendungen zu gering ist.

In der Druckschrift US 6,293,966 B1 wird ein Stent mit radioopaken Markerelementen offenbart, der an seinen distalen bzw. proximalen Enden C-förmig ausgebildete Elemente aufweist, die jeweils eine im Wesentlichen kugelförmige Aufnahme ausbilden. In diese Aufnahmen werden Markerelemente mit kugelförmigen Endabschnitten eingesetzt. Die kugelförmigen Endabschnitte werden formschlüssig und gegebenenfalls mittels einer Schweißverbindung in den durch die C-förmigen Elemente gebildeten Aufnahmen befestigt.

Die Druckschrift DE 698 36 656 T2 beschreibt einen bioabsorbierbaren Marker mit röntgendichten Bestandteilen zur Verwendung auf einer implantierbaren Endoprothese, wie einem Stent. Die bioabsorbierbaren röntgendichten Marker weisen beispielsweise poröse Abschnitte auf, die mit röntgendichtem Material gefüllt sind. Außerdem wird ein Marker beschrieben, der hohle, hohlraumartige und poröse Abschnitte aufweist, die mit röntgendichtem Material gefüllt sind. Außerdem zeigt der Stand der Technik einen Marker, der als längliches Element wie einem Filament ausgebildet ist, welches um Teile der implantierbaren Endoprothese geschlungen wird.

Die Druckschrift EP 2 399 619 A2 beschreibt ein Implantat mit einem Grundkörper und mit mindestens einem an dem Grundkörper befestigten Funktionselement das radioopakes und/oder röntgenopakes material umfasst. Das Funktionselement weist, mindestens im Bereich seiner Oberfläche, an der es mit dem Grundkörper verbunden ist, eine erste Schicht auf, welche überwiegend mindestens ein Metalloxid enthält. Das Funktionselement weist einen Mantel umfassend ein Metall oder eine Metallegierung enthaltend mindestens ein Metall der Gruppe mit den Elementen Wolfram, Tantal und Titan sowie einen Kern umfassend vorzugsweise ein Metall oder eine Metalllegierung enthaltend mindestens ein Metall der Gruppe mit den Elementen Platin und Gold auf. Ferner ist das Einbringen (Nieten, Laser- beziehungsweise Elektronenstrahlverschweißen) von Markern in Form von Ronden beziehungsweise Pellets (Durchmesser <400 μm) technologisch sehr anspruchsvoll und bedarf aufwändiger Handhabungstechnik. Bei Beaufschlagung von lokalen Werkstoffverbunden, wie sie im Falle einer Schweißverbindung vorliegen, kommt es zum Effekt des sogenannten Stress Shieldings; das heißt je größer der Unterschied der jeweiligen E-Module der verbundenen metallischen Werkstoffe ist, umso größer werden die mechanischen Spannungen an den Grenzflächen bei Biege- und oder Torsionsbeanspruchung. Diese beruhen auf dem unterschiedlichen Verformungsverhalten der Werkstoffe bei mechanischer Belastung. Dies kann bis zum Versagen des Werkstoffverbundes und damit zum Verlust des Markers führen.

## Zusammenfassung der Erfindung

Ein oder mehrere Nachteile des Standes der Technik werden mit Hilfe der erfindungsgemäßen Röntgenmarker gelöst oder zumindest gemindert. Der Röntgenmarker umfasst dabei einen Hohlzylinder aus einem ersten radioopaken Metall und ein mit dem Hohlzylinder fest verbundenes Markerelement, das im Innern des Hohlzylinders angeordnet ist. In einer bevorzugten Ausführungsform besteht das Markerelement aus einem zweiten radioopaken Metall oder einer Legierung daraus. Die für den Hohlzylinder und das weitere Markerelement verwendeten Metalle unterscheiden sich somit. Durch die Verwendung von unterschiedlichen Metallen können Lokalelemente bereits durch geschickte Materialwahl stark reduziert werden. Wird zum Beispiel das Hohlzylindermaterial dem Endoprothesenmaterial chemisch ähnlich gewählt, können Lokalelementeffekte bei metallischem Kontakt zum Körper der Endoprothese minimiert werden. Die Endoprothese ist allerdings ferner dadurch gekennzeichnet, dass sich zwischen dem Markerelement und dem Hohlzylinder ein elektrisch nicht leitfähiges Material befindet. Auf diese Weise lassen sich unerwünschte elektrochemische Prozesse zwischen den Metallen des Hohlzylinders und des Markerelements wirksam verhindern.

Die erfindungsgemäßen Röntgenmarker können somit mit verschiedenen Dimensionen vorgefertigt werden, auf Lager gelegt und bei Bedarf in verschiedene Endoprothesen eingebracht werden, sowie auch auf verschiedene Patientengrößen angepasst werden, da allein aufgrund unterschiedlicher Körpermassen und damit verbundener Eindringtiefen mehr oder weniger Röntgenstrahlung absorbiert wird. Während der Lagerung sind korrosive Prozesse zwischen den verschiedenen metallischen Komponenten des Markers durch die Trennung mittels des nicht leitfähigen Materials unterbunden, selbst wenn Feuchtigkeit eintritt. Auch in-vivo wird ein solches korrosives Verhalten wirkungsvoll verhindert. Des Weiteren bietet der hierin vorgeschlagene Röntgenmarker die Möglichkeit die Röntgensichtbarkeit nicht nur an Patienten, sondern auch an den zukünftigen Gerätestandard der Röntgengeräte anzupassen und somit auch in Zukunft maßgeschneiderte Röntgenmarker zur Verfügung zu stellen, sowohl durch Materialwahl als auch durch effektive Materialdichte. Auch ist es von Vorteil, dass ansonsten notwendige und kostenintensive Vergoldungsprozesse wegfallen, die häufig auch bei nicht resorbierbaren Metallen durchgeführt werden müssen.

Ein Hohlzylinder im Sinne der vorliegenden Erfindung ist nicht auf eine klassische Form eines geraden Rohres mit einer kreisrunden Grundfläche beschränkt. Ein Hohlzylinder kann jedwede Grundfläche einnehmen, einschließlich kreisrunder, ellipsenförmiger, ellipsoider, dreieckiger, quadratischer, rechteckiger, vieleckiger, achtförmiger oder auch vollkommen ungeordneter Formen sofern mindestens eine Fläche durch die umgebenen Wände eingeschlossen wird. Es ist ebenfalls denkbar, dass die zwei sich gegenüber liegenden Seiten, die den Hohlraum beschreiben eine unterschiedliche Form aufweisen. Ebenfalls ist es denkbar, dass die Verbindung zwischen den Grundflächen, die den Hohlraum definiert, nicht rechtwinkelig auf den Grundflächen ansetzt und dass die sich gegenüberliegenden Grundflächen nicht parallel zu einander liegen. Ein Hohlzylinder beschreibt vielmehr einen oder mehrere durchgängige Volumina, die vorzugsweise schlauchförmig ausgestaltet sind.

Ein Markerelement im Sinne der vorliegenden Erfindung kann jede erdenkliche Form annehmen mit der Maßgabe, dass das Markerelement in das Volumen des Hohlzylinders eingefügt werden kann. Dabei können so ungeordnete Formen wie Pulverpartikel angenommen werden wie auch Vollzylinder, die passgenau in den Hohlzylinder eingepasst werden können, wie auch kugelförmige Partikel.

Gemäß einer bevorzugten Variante liegt das Markerelement als Metallpulver oder in Form von Metallpartikeln vor. Das Metallpulver beziehungsweise die Metallpartikel sind dabei in das elektrisch nicht leitfähige Material eingebettet. Zur Herstellung des Röntgenmarkers kann demnach einfach ein Komposit aus Metallpulver beziehungsweise -partikel und elektrisch nicht leitfähigem Material in das Innere des Hohlzylinders eingebracht werden. Ein Gewichtsanteil des Metalls am Komposit sollte vorzugsweise im Bereich von 45 bis 97%, bevorzugt 60 bis 93% liegen. Das Markerelement ist dabei in dem Komposit so eingebettet, dass es zu keinem Metall-Metall-Kontakt zwischen Markerelement und Hohlzylinder kommt. Dies ist gut zu erreichen, wenn der Gewichtsanteil des Metalls am Komposit 97% nicht überschreitet und das Komposit vor dem Einsetzen in den Zylinder stark durchmischt wurde. Durch die starke Durchmischung wird gewährleistet, dass die Metallpulverkörner oder Metallpartikel vom elektrisch nicht leitfähigen Material umschlossen werden. Durch die gute Durchmischung und die elektrische Isolation der Metallpulverkörner oder Metallpartikel kann das Markerelement verfahrenstechnisch günstig auch ohne weitere Schritte in den Hohlzylinder eingefüllt werden, wenn die Konsistenz des Komposits dies gut zulässt. Eine elektrische Isolation zwischen Hohlzylinder und Markerelement kann bevorzugt auch dadurch erreicht werden, dass das nicht leitende Material entweder vor Einsetzen des Markerelements auf die Außenseite des Markerelements oder auf die Innenseite des Hohlzylinders aufgetragen wird.

Gemäß einer weiteren bevorzugten Variante ist das Markerelement ein Vollzylinder mit einem geringeren Durchmesser als der Innendurchmesser des Hohlzylinders. Das elektrisch nicht leitfähige Material bildet dabei eine Schicht zwischen einer inneren Mantelfläche des Hohlzylinders und einer Mantelfläche des Vollzylinders. Der Vollzylinder gewährt eine hinreichend hohe Röntgensichtbarkeit. Die umlaufende Schicht weist insbesondere eine Schichtdicke im Bereich von 7,5 - 15 μm auf. In diesem Schichtdickenbereich wird bevorzugt eine effektive elektrische Isolation, sowie eine gute Haftung des Vollzylinders an den Hohlzylinder erreicht ohne die Ausmaße des Röntgenmarkers unnötig zu vergrößern.

Zur Verbesserung der Haftung weist die innere Mantelfläche des Hohlzylinders und die Mantelfläche des Vollzylinders vorzugsweise einen Rauheitswert Ra im Bereich von 0,4 - 2 μm, weiter bevorzugt von 0,6 bis 1,2 μm auf. Eine gute Anhaftung kann dazu beitragen, dass selbst bei geringer Wanddicke die Kräfte, die den Röntgenmarker bei mechanischer Belastung aus dem Verbund mit der Endoprothese heraus lösen würden, hoch sind und somit eine hohe Verbundfestigkeit generiert wird. Die Wahrscheinlichkeit für den ungewollten Verlust des Markers wird dadurch deutlich herabgesetzt.

Ferner ist bevorzugt, wenn der Hohlzylinder aus Wolfram, Tantal oder Legierungen dieser Metalle besteht. Weiterhin kann der Hohlzylinder einen Durchmesser im Bereich von 100 - 500 μm und eine Höhe im Bereich von 50 - 180 μm aufweisen. Bevorzugt ist zudem, wenn der Hohlzylinder eine Wanddicke im Bereich von 10 - 50 μm aufweist. Mit diesen Ausmaßen und Materialien kann eine ausreichend gute Röntgensichtbarkeit erreicht werden, ohne unnötigen Materialaufwand in Kauf nehmen zu müssen.

Das elektrisch nicht leitfähige Material ist vorzugsweise ein polymerer Klebstoff. Geeignete polymere Klebstoffe umfassen vorzugsweise Polyurethane, Silikone, Polymethylmethacrylate, Cyanoacrylate, Polyester und Epoxidharze.

In einer bevorzugten Ausführungsform werden elastische polymere Klebstoffe verwendet. Geeignete elastische Klebstoffe umfassen Silikone, Polylactide, Polyhydroxybuttersäure sowie deren Blends. Elastische Klebstoffe haben den Vorteil, dass sie zu einer verbesserten Trackability, also zur Anpassung an das umgebende Gewebe während des Vordringens der Endoprothese beim Einsetzen, beitragen. Ein vorzeitiger Verlust eines Röntgenmarkers wird dadurch nochmals unwahrscheinlicher.

Das Markerelement, insbesondere in der Ausführungsform eines Vollzylinders kann insbesondere aus Gold, Platin, Iridium oder Legierungen daraus bestehen, also Metallen, die besonders gute radioopake Eigenschaften besitzen, jedoch auch kostenintensiv sind. Wenn der Hohlzylinder aus Tantal oder seinen Legierungen geformt ist, besteht das Markerelement vorzugsweise aus Wolfram. Durch die kompakte Bauweise unter gleichzeitiger Isolation zur Verhinderung von Lokalelementen kann hier kostenschonend ein Röntgenmarker aufgebaut werden.

In einer bevorzugten Ausführungsform kann durch geeignete Wahl der Materialien für Hohlzylinder und Markerelement in vorteilhafter Weise ein besonders breiter Energiebereich von Röntgenstrahlen absorbiert werden. In einer bevorzugten Ausführungsform ist der Hohlzylinder aus Tantal oder Legierungen daraus gefertigt und das Markerelement aus Gold oder Platin hergestellt. In einer weiteren bevorzugten Ausführungsform sind der Hohlzylinder aus Wolfram und das Markerelement aus Gold gefertigt. Insbesondere ist bevorzugt, wenn der Hohlzylinder aus Tantal und das Markerelement aus Gold gefertigt ist.

Ein weiterer Aspekt der Erfindung liegt in der Bereitstellung einer Endoprothese mit einem metallischen Grundkörper und mit mindestens einem an dem Grundkörper befestigten erfindungsgemäßen Röntgenmarker. Vorzugsweise besteht der Grundkörper aus einem biodegradierbaren, metallischen Werkstoff, insbesondere einer Magnesiumlegierung. Zwischen dem Röntgenmarker und dem Grundkörper befindet sich vorzugsweise ein elektrisch nicht leitfähiges Material, insbesondere ein polymerer Klebstoff, der korrosive Prozesse zwischen den beiden metallischen Werkstoffen verhindert. Ein Montageprozess kann durch Einkleben vorgefertigter Marker in vorgegebene Aufnahmen an der Endoprothese vereinfacht werden.

In einer bevorzugten Ausführungsform kann das elektrisch nicht leitfähige Material in Form einer Schicht zwischen Hohlzylinder und Endoprothese ausgebildet sein. In einer weiter bevorzugten Ausführungsform hat die Schicht aus elektrisch nicht leitfähigem Material eine Dicke von über 5 μm und bevorzugt eine Dicke von 7,5 - 15 μm.

Es hat sich vorteilhafter Weise gezeigt, dass durch Einbringung von zwei isolierenden Schichten, wobei die eine zwischen Markerelement und Hohlzylinder und die zweite zwischen Hohlzylinder und Endoprothese angeordnet ist, eine vollständige oder annähernd vollständige Unterbindung der Entstehung von galvanischen Elementen und damit verbundenen Stromflüssen durch Lokalelementbildung erreicht werden kann. Eine solche Endoprothese weist dadurch eine wesentlich verbesserte Lebensdauer und Verträglichkeit auf, sowie ein vermindertes Risiko vorzeitig einen Röntgenmarker unkontrolliert zu verlieren. Ferner können diese Marker in Endoprothesen unterschiedlichster Materialien eingesetzt werden, ohne dass die Gefahr besteht, dass es zu negativen Interaktionen zwischen den verwendeten Materialien kommt.

In einer weiteren Ausführungsform wird eine Endoprothese mit einem metallischen Grundkörper und mit mindestens einem an dem Grundkörper befestigten erfindungsgemäßen Röntgenmarker vorgeschlagen, wobei der metallische Grundkörper zumindest am proximalen und distalen Ende einen Mehrfachmarker, insbesondere einen Doppelmarker aufweist. Ein Mehrfachmarker, insbesondere ein Doppelmarker, umfasst hierbei mindestens zwei benachbarte unterschiedliche erfindungsgemäße Röntgenmarker, im Falle eines Doppelmarkers zwei unterschiedliche erfindungsgemäße Röntgenmarker. Die unterschiedlichen erfindungsgemäßen Röntgenmarker unterscheiden sich hierbei durch die Wahl der Materialien für den Hohlzylinder und das Markerelement, insbesondere aber durch die Wahl für das Material des Markerelements. Somit wird eine Endoprothese vorgeschlagen, mit einem metallischen Grundkörper und mit mindestens einem an dem Grundkörper befestigten erfindungsgemäßen Röntgenmarker, wobei der metallische Grundkörper zumindest am proximalen und distalen Ende einen Mehrfachmarker aufweist, wobei der Mehrfachmarker mindestens zwei benachbarte unterschiedliche erfindungsgemäße Röntgenmarker umfasst, und vorzugsweise wobei die mindestens zwei benachbarten unterschiedlichen erfindungsgemäßen Röntgenmarker unterschiedliche Markerelemente aufweisen.

In einer beispielshaften Ausführungsform umfasst eine solche hier vorgeschlagene Endoprothese je einen Doppelmarker sowohl am distalen als auch am proximalen Ende, wobei die Hohlzylinder eines Doppelmarkers aus Tantal gefertigt sind, während die Markerelemente aus Wolfram und aus Platin oder in einer alternativen Ausführungsform aus Gold und Platin gefertigt sind.

Eine solche Ausführungsform einer Endoprothese, umfassend Mehrfachmarker wie hierin beschrieben hat den Vorteil, dass gewährleistet ist, dass mit unterschiedlichen Röntgengeräten eine optimale Röntgensichtbarkeit generiert werden kann. So führt beispielsweise die Gerätespezifik (mit einem bestimmten Beschleunigungsspannungsbereich xxx) des Röntgengerätes yyy zu einer besseren Sichtbarkeit des Wolframmarkers. Ein anderes Röntgengerät zzz (mit anderem Beschleunigungsspannungsbereich aaa) stellt die Marker aus Gold und/ oder Platin besser dar. Damit wird eine Geräteunabhängigkeit für die Röntgensichtbarkeit hergestellt.

Somit kann gemäß dem vorliegenden Vorschlag eine Endoprothese bereitgestellt werden, die einen hierin vorgeschlagenen Schutz vor Lokalelementen aufweist, und darüber hinaus durch eine optimale Röntgensichtbarkeit ausgezeichnet ist.

Weitere bevorzugte Ausgestaltungen der Erfindung ergeben sich aus der nachfolgenden Beschreibung.

## Kurzbeschreibung der Figuren

Die Erfindung wird nachfolgend anhand von in Figuren dargestellten Ausführungsbeispielen näher erläutert. Es zeigen:

Fig. 1    Eine schematische Draufsicht auf einen Röntgenmarker gemäß einer ersten erfindungsgemäßen Ausführungsform, der in einer komplementären Aufnahme einer Endoprothese sitzt.

Fig. 2 Eine schematische Draufsicht auf einen Röntgenmarker gemäß einer zweiten erfindungsgemäßen Ausführungsform, der in einer komplementären Aufnahme einer Endoprothese sitzt.

Fig. 3 Schnittansicht durch den in Figur 2 dargestellten Röntgenmarker.

**Detaillierte Beschreibung der Erfindung**

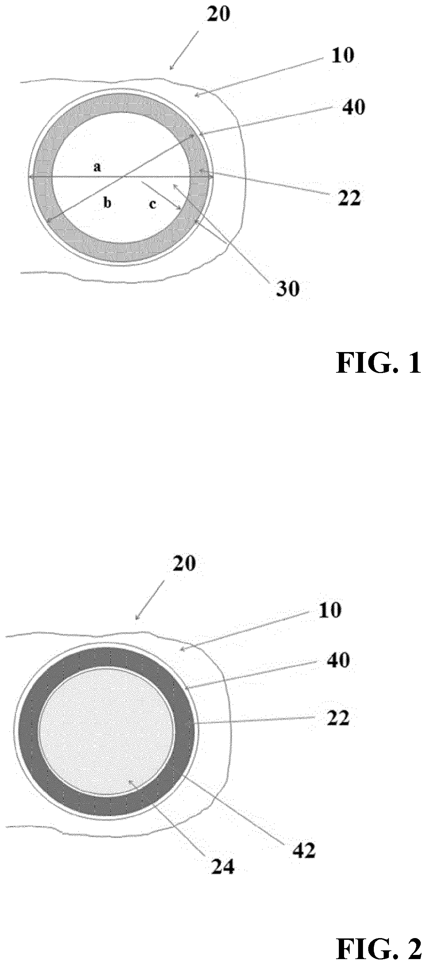

Figur 1 zeigt einen stark vergrößerten Abschnitt eines Grundkörpers 10 einer Endoprothese, insbesondere eines Stents. Der hier nicht weiter dargestellte Grundkörper 10 weist in der Regel zick-zack- oder mäanderförmig gefaltete, im Wesentlichen in Umfangsrichtung verlaufende oder helixförmige Stege als Stützelemente sowie im Wesentlichen in Längsrichtung des Stents verlaufende Stege als Verbindungselemente auf. Der in Figur 1 dargestellte Abschnitt ist ein Teil eines Strukturelements, das der Aufnahme eines Röntgenmarkers 20 dient. Der Grundkörper 10 besteht beispielsweise aus einer biodegradierbaren Magnesiumlegierung. Die runde Aufnahme am Gerüst hat einen Durchmesser a, beispielsweise von ca. 350 $\mu$m und weist eine Stegwandstärke auf, beispielsweise von ca. 120 $\mu$m Der in Figur 1 dargestellte Röntgenmarker 20 weist einen Hohlzylinder 22 aus Tantal auf, dessen Inneres mit einem Komposit 30 aus einem Markerelement und einem elektrisch nicht leifähigem Material befüllt ist. Das Markerelement ist dabei in dem Komposit so eingebettet, dass es zu keinem Metall-Metall-Kontakt zwischen Markerelement und Hohlzylinder kommt. Der Röntgenmarker 20 ist durch eine isolierende Klebstoffschicht 40 mit dem Grundgerüst 10 verbunden.

Der Röntgenmarker 20 kann wie folgt hergestellt werden:

Ein Rohrabschnitt aus 99%-igem Reintantal wird auf eine geeignete Länge geschnitten, beispielsweise von 150 $\mu$m. Ein Außendurchmesser b des Rohrabschnitts b kann beispielsweise 310 $\mu$m betragen, die Wandstärke c des Rohrs kann ferner beispielsweise 50 $\mu$m betragen. Der resultierende Innendurchmesser in dem hier beschriebenen Beispiel liegt somit bei 210 $\mu$m. Die Innen- und Außenoberflächen sind sandgestrahlt, anschließend thermisch oxidiert und weisen einen geeigneten Rauheitswert von beispielsweise Ra = 0,8 $\mu$m auf.

Es folgt die Herstellung eines Metallpulver/Polymercompounds mit folgenden Inhaltsstoffen im Verhältnis von 92 Gew.% Metallpulver und 8 Gew.% Polymer:

Metallpulver: Au, mittlere Partikelgröße 15 nm

Polymer: Silikonklebstoff MED2-4213 (Hersteller Fa. NUSIL Technology)

Nach dem Abwägen der Einzelkomponenten, folgt manuelles Verrühren und anschließendes Vermengen der Komponenten in Zentrifugen zur Herstellung einer homogenen Mischung. Die Mischung wird in Kartuschen verfüllt und über eine Kanüle ohne Einschlüsse von Luft in das Tantalrohr injiziert. Anschließend wird in einer Temperierkammer bei 150°C über 15 min ausgehärtet.

Mehrere dieser nunmehr gefüllten Rohrabschnitte mit dem ausgehärteten Inlay werden aufrecht in eine Schablone verbracht und beidseitig auf die Nennhöhe von 120 $\mu$m auf Schleifscheiben heruntergeschliffen.

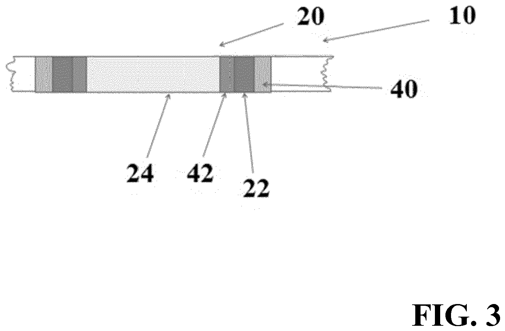

Den Figuren 2 und 3 ist eine weitere Ausführungsform des Röntgenmarkes 20 zu entnehmen. Grundgerüst 10 und Klebstoffschicht 40 können analog der in Figur 1 dargestellten Ausführungsform ausgestaltet werden, wobei hier auch alternative Ausmaße angewendet werden können. So kann zum Beispiel abweichend zum Ausführungsbeispiel der Fig. 1 der Durchmesser der Aufnahme 450 $\mu$m betragen und das Grundgerüst 10 eine Stegwandstärke von 100 $\mu$m aufweisen. Der Hohlzylinder 22 besteht hier aus Wolfram; er kann aber auch aus anderen geeigneten Materialien hergestellt sein. Im Innern des Hohlzylinders 22 bildet ein Vollzylinder 24 ein weiteres Markerelement. Der Vollzylinder 24 hat einen geringeren Durchmesser als der Innendurchmesser des Hohlzylinders 22. Der sich ergebende Zwischenraum ist mit einem elektrisch nicht leitfähigen Material, das eine Schicht 42 bildet, befüllt.

Der Röntgenmarker 20 kann wie folgt hergestellt werden:

Es wird ein Rohrabschnitt aus 99%-igem Reinwolfram auf eine Länge von 130 $\mu$m geschnitten. Ein Außendurchmesser des Rohrabschnitts beträgt 410 $\mu$m, die Wandstärke beträgt 80 $\mu$m. Der resultierende Innendurchmesser liegt bei 250 $\mu$m. Die Innen- und Außenoberflächen sind sandgestrahlt, anschließend thermisch oxidiert und weisen einen geeigneten Rauheitswert von beispielsweise Ra = 0,8 $\mu$m auf.

Ferner wird eine Ronde (Vollzylinder) aus Reingold (99%) mit einem Außendurchmesser von 220 $\mu$m und einer Höhe von 130 $\mu$m bereitgestellt. Die Ronde wird in eine Polymerlösung des Silikonklebstoffs MED2-4213 (Hersteller Fa. NUSIL Technology) getaucht und anschließend in den Wolframrohrabschnitt verbracht. Es folgt Aushärten in einer Temperierkammer bei 150°C über 15 min. Mehrere dieser gefüllten Rohrabschnitte werden aufrecht in eine Schablone verbracht und beidseitig auf die Nennhöhe von 120 $\mu$m auf Schleifscheiben heruntergeschliffen.

Gemäß einer weiteren Variante für die vorab beschriebene Ausführungsform kann das Grundgerüst aus Nitinol, der Röntgenmarker mit einem Hohlzylinder aus Tantal und einer Ronde aus Platin bestehen. Alternativ kann das Grundgerüst aus CoCr (L605), der Röntgenmarker mit einem Hohlzylinder aus Wolfram und einer Ronde aus Gold bestehen.

**Patentansprüche**

1. Röntgenmarker (20) für eine Endoprothese, wobei der Röntgenmarker (20) einen Hohlzylinder (22) aus einem ersten radiopaken Metall und ein mit dem Hohlzylinder (22) fest verbundenes Markerelement umfasst, das im Innern des Hohlzylinders (22) angeordnet ist und aus einem zweiten radiopaken Metall oder einer Legierung daraus besteht, **dadurch gekennzeichnet, dass** sich zwischen dem Markerelement und dem Hohlzylinder (22) ein elektrisch nicht leitfähiges Material befindet.

2. Röntgenmarker nach Anspruch 1, wobei das Markerelement als Metallpulver oder in Form von Metallpartikeln vorliegt, das/die in das elektrisch nicht leitfähige Material eingebettet sind.

3. Röntgenmarker nach Anspruch 1, wobei das Markerelement ein Vollzylinder (24) mit einem geringeren Durchmesser als der Innendurchmesser des Hohlzylinders (22) ist und das elektrisch nicht leitfähige Material eine Schicht (42) zwischen einer inneren Mantelfläche des Hohlzylinders (22) und einer Mantelfläche des Vollzylinders (24) bildet.

4. Röntgenmarker nach Anspruch 3, wobei die umlaufende Schicht (42) eine Schichtdicke im Bereich von 7,5 - 15 $\mu$m aufweist.

5. Röntgenmarker nach Anspruch 3 oder 4, wobei die innere Mantelfläche des Hohlzylinders (22) und die Mantelfläche des Vollzylinders (24) eine Rauheit Ra im Bereich von 0,4 - 2 $\mu$m aufweisen.

6. Röntgenmarker nach einem der vorherigen Ansprüche, wobei der Hohlzylinder (22) aus Wolfram, Tantal oder Legierungen dieser Metalle besteht.

7. Röntgenmarker nach einem der vorherigen Ansprüche, wobei der Hohlzylinder (22) einen Durchmesser im Bereich von 100 - 500 $\mu$m und eine Höhe im Bereich von 50 - 180 $\mu$m aufweist.

8. Röntgenmarker nach einem der vorherigen Ansprüche, wobei der Hohlzylinder (22) eine Wanddicke im Bereich von 10 - 50 $\mu$m aufweist.

9. Röntgenmarker nach einem der vorherigen Ansprüche, wobei das elektrisch nicht leitfähige Material ein polymerer Klebstoff ist.

10. Röntgenmarker nach einem der vorherigen Ansprüche, wobei das Markerelement aus Gold, Platin, Iridium oder Legierungen daraus besteht.

11. Röntgenmarker nach einem der Ansprüche 1 bis 9, wobei das Markerelement aus Wolfram besteht und der Hohlzylinder (22) aus Tantal oder Legierungen daraus geformt ist.

12. Endoprothese mit einem metallischen Grundkörper (10) und mit mindestens einem an dem Grundkörper (10) befestigten Röntgenmarker (20) gemäß einem der vorhergehenden Ansprüche.

13. Endoprothese nach Anspruch 12, wobei der Grundkörper (10) aus einem biodegradierbaren, metallischen Werkstoff besteht.

14. Endoprothese nach Anspruch 12 oder 13, wobei sich zwischen dem Röntgenmarker (20) und dem Grundkörper (10) ein elektrisch nicht leitfähiges Material befindet.

15. Endoprothese nach einem der Ansprüche 12 bis 14, wobei der metallische Grundkörper (10) zumindest an einem proximalen und einem distalen Ende einen Mehrfachmarker aufweist, wobei der Mehrfachmarker mindestens zwei benachbarte unterschiedliche Röntgenmarker (20) umfasst.

**Claims**

1. An x-ray marker (20) for an endoprosthesis, wherein the x-ray marker (20) comprises a hollow cylinder (22) made of a first radiopaque metal and a marker element, which is fixedly connected to the hollow cylinder (22) and which is arranged inside the hollow cylinder (22) and consists of a second radiopaque metal or an alloy thereof, **characterized in that** an electrically non-conductive material is located between the marker element and the hollow cylinder (22).

2. The x-ray marker as claimed in claim 1, wherein the marker element is present as metal powder or in the form of metal particles which is/are embedded in the electrically non-conductive material.

3. The x-ray marker as claimed in claim 1, wherein the marker element is a solid cylinder (24) with a diameter smaller than the inner diameter of the hollow cylinder (22) and the electrically non-conductive material forms a layer (42) between an inner lateral surface of the hollow cylinder (22) and a lateral surface of the solid cylinder (24).

4. The x-ray marker as claimed in claim 3, wherein the peripheral layer (42) has a layer thickness in the range of 7.5-15 μm.

5. The x-ray marker as claimed in claim 3 or 4, wherein the inner lateral surface of the hollow cylinder (22) and the lateral surface of the solid cylinder (24) have a roughness Ra in the range of 0.4-2 μm.

6. The x-ray marker as claimed in any one of the preceding claims, wherein the hollow cylinder (22) consists of tungsten, tantalum or alloys of these metals.

7. The x-ray marker as claimed in any one of the preceding claims, wherein the hollow cylinder (22) has a diameter in the range of 100-500 μm and a height in the range of 50-180 μm.

8. The x-ray marker as claimed in any one of the preceding claims, wherein the hollow cylinder (22) has a wall thickness in the range of 10-50 μm.

9. The x-ray marker as claimed in any one of the preceding claims, wherein the electrically non-conductive material is a polymer adhesive.

10. The x-ray marker as claimed in any one of the preceding claims, wherein the marker element consists of gold, platinum, iridium or alloys thereof.

11. The x-ray marker as claimed in any one of claims 1 to 9, wherein the marker element consists of tungsten and the hollow cylinder (22) is formed from tantalum or alloys thereof.

12. An endoprosthesis having a metallic main body (10) and having at least one x-ray marker (20) as claimed in any one of the preceding claims secured to the main body (10).

13. The endoprosthesis as claimed in claim 12, wherein the main body (10) consists of a biodegradable, metallic material.

14. The endoprosthesis as claimed in claim 12 or 13, wherein an electrically non-conductive material is located between the x-ray marker (20) and the main body (10).

15. The endoprosthesis as claimed in any one of claims 12 to 14, wherein the metallic main body (10) has a multiple marker at least at a proximal and a distal end, wherein the multiple marker comprises at least two adjacent different x-ray markers (20).

**Revendications**

1. Marqueur aux rayons X (20) pour une endoprothèse, où le marqueur aux rayons X (20) comprend un cylindre creux (22) à base d'un premier métal radio-opaque et un élément de marquage, relié solidement avec le cylindre creux (22), qui est disposé à l'intérieur du cylindre creux (22) et qui à base d'un deuxième métal radio-opaque ou est constitué d'un alliage de celui-ci,
**caractérisé en ce**
**qu'**entre l'élément de marquage et le cylindre creux (22), il y a un matériau non conducteur électriquement.

2. Marqueur aux rayons X selon la revendication 1, dans lequel l'élément de marquage se présente sous la forme de poudre métallique ou de particules métalliques, qui est ou sont incorporée(s) dans le matériau non conducteur électriquement.

3. Marqueur aux rayons X selon la revendication 1, dans lequel l'élément de marquage est un cylindre plein (24) avec un diamètre inférieur au diamètre intérieur du cylindre creux (22) et le matériau non conducteur électriquement forme une couche (42) entre une surface d'enveloppe intérieure du cylindre creux (22) et une surface d'enveloppe du cylindre plein (24).

4. Marqueur aux rayons X selon la revendication 3, dans lequel la couche (42) circonférentielle présente une épaisseur de couche dans la plage de 7,5 à 15 μm.

5. Marqueur aux rayons X selon la revendication 3 ou 4, dans lequel la surface d'enveloppe intérieure du cylindre creux (22) et la surface d'enveloppe du cylindre plein (24) présentent une rugosité Ra dans la plage de 0,4 à 2 μm.

6. Marqueur aux rayons X selon l'une des revendications précédentes, dans lequel le cylindre creux (22) est constitué de tungstène, de tantale ou d'alliages de ces métaux.

7. Marqueur aux rayons X selon l'une des revendications précédentes, dans lequel le cylindre creux (22) présente un diamètre dans la plage de 100 à 500 μm et une hauteur dans la plage de 50 à 180 μm.

8. Marqueur aux rayons X selon l'une des revendications précédentes, dans lequel le cylindre creux (22) présente une épaisseur de paroi dans la plage de 10 à 50 μm.

9. Marqueur aux rayons X selon l'une des revendications précédentes, dans lequel le matériau non conducteur électriquement est un adhésif polymère.

10. Marqueur aux rayons X selon l'une des revendications précédentes, dans lequel l'élément de marquage est constitué d'or, de platine, d'iridium ou d'alliages de ceux-ci.

11. Marqueur aux rayons X selon l'une des revendications 1 à 9, dans lequel l'élément de marquage est constitué de tungstène et le cylindre creux (22) est formé à base de tantale ou d'alliages de celui-ci.

12. Endoprothèse avec un corps de base (10) métallique et avec au moins un marqueur aux rayons X (20) fixé sur le corps de base (10) selon l'une des revendications précédentes.

13. Endoprothèse selon la revendication 12, dans laquelle le corps de base (10) est constitué d'un matériau métallique biodégradable.

14. Endoprothèse selon la revendication 12 ou 13, dans laquelle un matériau non conducteur électriquement se trouve entre le marqueur aux rayons X (20) et le corps de base (10).

15. Endoprothèse selon l'une des revendications 12 à 14, dans laquelle le corps de base (10) métallique présente au moins un marqueur multiple à une extrémité proximale et à une extrémité distale, où le marqueur multiple comprend au moins deux marqueurs aux rayons X (20) différents voisins.

**20**

**10**

**40**

a

**22**

b c

**30**

## FIG. 1

**20**

**10**

**40**

**22**

**42**

**24**

## FIG. 2

**FIG. 3**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2184038 A2
- US 20070043429 A1
- EP 1570808 B1
- US 6355058 B1
- US 6293966 B1
- DE 69836656 T2
- EP 2399619 A2